# EUROPEAN PATENT SPECIFICATION

(11) **EP 1 922 061 B1**
(45) Date of publication and mention of the grant of the patent: **17.12.2008**
(21) Application number: 06761981.7
(22) Date of filing: 07.06.2006
(51) Int. Cl.: A61K 9/16, A61K 31/4439

(54) **ORAL SOLID PHARMACEUTICAL FORMULATION OF THE TRIBULIN INHIBITOR INDIBULIN**
FESTE ORALE DARREICHUNGSFORM DES TRIBULINHEMMSTOFFS INDIBULIN
FORMULATION SOLIDE PHARMACEUTIQUE ORALE DU COMPOSE INDIBULINE INHIBITEUR DE LA TUBULINE

(30) Priority: 14.06.2005 US 151459
(43) Date of publication of application: 21.05.2008
(73) Proprietor: ZIOPHARM ONCOLOGY, INC., Boston, MA 02129 (US)
(72) Inventor: ROESSLER, Berthold, 33790 Halle (DE); RAAB, Gerhard, 63549 Ronneburg (DE); REISSMANN, Thomas, 21335 Lueneburg (DE)
(74) Representative: Hock, Joachim
(86) International application number: PCT/EP2006/005423
(87) International publication number: WO 2006/133835

(56) References cited:
- WO-A-03/037861
- WO-A-20/06052712
- WO-A1-20/06002887

## Description

### FIELD OF THE INVENTION

The present invention relates to a specific pharmaceutical formulation for oral administration of the poorly soluble and therefore hardly bioavailable tubulin inhibitor Indibulin and a process for its manufacture.

### BACKGROUND OF THE INVENTION

During mitosis, a cell's DNA is replicated and then divided into two new cells. The process of separating the newly replicated chromosomes into the two forming cells involves spindle fibers constructed with microtubules, which themselves are formed by long chains of smaller protein subunits called tubulins. Spindle microtubules attach to replicated chromosomes and pull one copy to each side of the dividing cell. Without these microtubules, cell division is not possible.

Microtubules therefore are among the most important sub-cellular targets of anticancer chemotherapeutics because they are present in all cells and are necessary for mitotic, interphase and cell maintenance functions (e.g. intracellular transport, development and maintenance of cell shape, cell motility, and possibly distribution of molecules on cell membranes). Compounds that interact with tubulin can interfere with the cell cycle by causing tubulin precipitation and sequestration, thereby interrupting many important biologic functions that depend on the microtubular class of subcellular organelles. Therefore, such compounds can potentially inhibit the proliferation of tumor cell lines derived from various organs. See, e.g., Bacher et al. (2001) Pure Appl. Chem. 73:9 1459-1464 and Rowinsky & Donehower (1991) Pharmac. Ther. 52:35-84.

Accordingly, new, synthetic, small-molecule chemical entities that bind to tubulin, but are neither a substrate of transmembrane pumps nor interfere with the function of axonal microtubules, would strongly increase the therapeutic index in the treatment of malignancies.

A series of synthetic molecules that bind to tubulin are currently being evaluated in the preclinical or early clinical stage. Among them is the synthetic compound, *N*-(pyridine-4-yl)-[1-(4-chlorobenzyl)-indole-3-yl]glyoxylic acid amide, named Indibulin (INN) having the formula C₂₂H₁₆ ClN₃O₂ and the following structure:

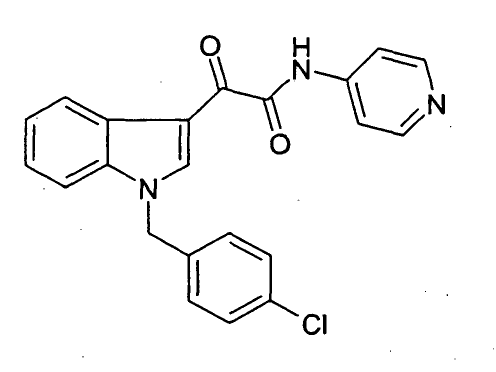

Indibulin is a synthetic stall molecule tubulin inhibitor with significant antitumor activity *in vitro* and *in vivo*. It destabilizes microtubules in tumor cells, as well as in a cell-free system. The binding site of Indibulin does not appear to overlap with the tubulin-binding sites of the well-characterized microtubule-destabilizing agents vincristine or colchicine. Furthermore, the molecule selectively blocks cell cycle progression at metaphase.

*In vitro*, Indibulin exerts significant antitumor activity against a variety of malignancies (e.g., prostate, brain, breast, pancreas, and colon). Indibulin displays high *invivo* antineoplastic efficacy in animals. Based on its mechanism of action it is expected to target all types of solid tumors. It is also expected to exhibit antiasthmatic, antiallergic, immuno-suppressant and immunomodulating actions. No neurological symptoms have so far been found in animal experiments. In preclinical experiments in rodents the compound was very well tolerated at highly effective doses. Another advantage for further development is, in contrast to other tubulin-inhibitory compounds, its easy synthesis.

Indibulin is obtained by chemical synthesis as a white crystalline powder. The solubility in hydrophilic solvents is poor, for example it is practically insoluble in water, methanol, ethanol or 2-propanol. Due to these properties, the bioavailability of pure Indibulin is very low. This is also valid for common pharmaceutical dosage forms of Indibulin, e.g. powder, granula, tablets or capsules.

In various organic solvents, for example dimethylformamide, dimethylsulfoxide and N-methylpyrrolidone it shows a sufficient solubility. But these organic solvents cannot be used for application in humans, due to their toxicity.

Highly concentrated (roughly > 50% w/v) organic acids, for example acetic acid or lactic acid are relatively good solvents for Indibulin.

For the improvement of the bioavailability of poorly soluble drugs various technologies are known and proven:
(i) Micronisation of the active ingredient and formulation to oral dosage forms, for example suspensions, capsules or tablets [lit.: R. Voigt, Lehrbuch der Pharm. Tech.; Hagers Handbuch Band 2, Kap. 12.2; Bauer, Frömming, Führer, Pharmazeutische Technologie]. However, these types of formulation lead in the case of Indibulin to a relatively low and insufficient bioavailability and therefore to low plasma levels and no efficacy.
(ii) Dissolution or suspension in organic solvents and surfactants. [lit.: R. Voigt, Lehrbuch der Pharm. Tech; Hagers Handbuch Band 2, Kap. 12.2; Bauer, Frömming, Führer, Pharmazeutische Technologie]. The use of surfactants leads to an increased bioavailability of Indibulin in animal tests, but in all cases the formulation were not acceptable for human use, due to the high amount of excipients needed.
(iii) Preparation of colloidal suspensions, nano- or microparticle suspensions. For example by using high shear forces the substance is crushed to nanoparticulate size or the substance is dissolved and afterwards precipitated out of a solvent mixture. For stabilization often surfactants and/or salts are added. Additionally the viscosity of the suspension can be modified to decrease sedimentation. [see also U.S. Pat. No. 4,826,689]. However, the manufacturing and processing of this type of pharmaceutical formulation is of extremely high complexicity.
(iv) Preparation of a drinking solution for Indibulin. For Indibulin it is additionally known that an oversaturated solution in lactic acid can be prepared and orally administered [see also DE 2004 031538.8]. This solution has to be freshly prepared prior to administration, due to stability reasons. Such a solution shows a good bioavailability of Indibulin, but due to the relatively high concentration of lactic acid (5 to 10 % w/v) the amount which can be administered is limited by taste and side effects. Since the concentration of the solution ready to use is approximately 1 mg/ml in 10% lactic acid, the applicable volume is limited to roughly 60 to 80 ml.

Therefore, a strong need exists for a new pharmaceutical Indibulin formulation which exhibits improved bioavailability of Indibulin without showing the disadvantages given in the prior art as mentioned above. Thus, it is an object of the present invention to provide a new pharmaceutical formulation exhibiting improved bioavailability of the pure Indibulin substance. It is a further object of the present invention to provide a respective method for the manufacture of such a pharmaceutical formulation.

### SUMMARY OF THE INVENTION

The present invention relates to a pharmaceutical formulation of Indibulin for oral administration comprising a granulate containing micronized Indibulin having a particle size of less than 20 µm for at least 99 vol.-% of the particles, at least one hydrophilic surfactant, and one or more additional capsulation excipients. Further, the present invention relates to a tablet prepared by using said pharmaceutical formulation and a capsule filled with said pharmaceutical formulation, respectively.

The pharmaceutical formulation of Indibulin according to the present invention is based on micronization of Indibulin combined with a granulation procedure using a hydrophilic surfactant (e.g. polysorbate, poloxamer, cremophor) and common capsulation excipients (e.g. cellulose, starch, highly disperse silicon dioxide, etc). This leads to a sufficient bioavailability and therefore effective plasma levels, which is a significant improvement in formulation of the poorly soluble drug Indibulin. Compared with an ordinary capsule or tablet made of micronized Indibulin the bioavailability as gained by the pharmaceutical formulation of Indibulin according to the present invention is significantly higher. It is on the same level as found for the drinking solution of lactic acid, but avoiding the disadvantages of that formulation with a limit of dosage.

Additionally, a process for manufacturing said pharmaceutical formulation, comprising the steps of micronizing Indibulin to a particle size of less than 20 µm for more than 99 vol.-% of the particles and homogeneously mixing the micronized Indibulin with at least one hydrophilic surfactant and additional capsulation excipients, is provided according to the present invention.

### DETAILED DESCRIPTION OF THE INVENTION

One aspect of the present invention relates to a pharmaceutical formulation of Indibulin for oral administration comprising a granulate containing micronized Indibulin having a particle size of less than 20 µm for at least 99 vol.-% of the particles, at least one hydrophilic surfactant, and one or more capsulation excipients. Preferably, the micronized Indibulin has a particle size of less than 10 µm for at least 90 vol.-% of the particles. More preferably, the micronized Indibulin has a particle size of less than 10 µm for at least 99 vol.-% of the particles. Even more preferred, the micronized Indibulin has a mean particle size in the range of 2 to 4 µm.

In a preferred embodiment of the present invention, the pharmaceutical formulation comprises Indibulin in an amount of about 10 to about 50 weight %, the at least one hydrophilic surfactant in an amount of about 1 to about 10 weight %, and one or more capsulation excipients in an amount of about 40 to about 80 weight %, the three constituents always adding up to 100 weight % of said pharmaceutical formulation.

The hydrophilic surfactant is not subject to any particular limitation as long as it is capable of acting as an oil-in-water surfactant. Preferably, the one or more hydrophilic surfactant(s) is/are selected from the group consisting of polysorbates, poloxamers, cremophors and polyalkylene glycols. Any type of polysorbate can be employed, but particularly the polysorbate is selected from polysorbate 20, polysorbate 40, polysorbate 60 or polysorbate 80, more preferred from polysorbate 80. Further, any type of poloxamers can be employed. Poloxamers are surfactant-like block polymers having a central polypropylene glycole moiety which on both terminal ends is connected to a macrogol moiety. Typical poloxamers suited for the present invention are poloxamers 188 and 407, particularly poloxamer 188. Cremophors are non-ionic emulsifiers obtained by causing ethylene oxide to react with castor oil particularly in a molar ratio of about 35 moles to 1 mole. Other common names are polyoxyethyleneglyceroltriricinoleate 35 or polyoxyl 35 castor oil. A typical cremophor is for example Cremophor^{®} EL supplied by BASF AG, Germany.

As capsulation excipients those which are common in the art can be suitably used in the present invention. In particular, those capsulation excipients can comprise cellulose such as microcristalline cellulose or a derivative thereof, gelatine, starch, particularly com starch, and highly disperse silicon dioxide (aerosil). Typically, the capsulation excipients comprise a mixture of microcrystalline cellulose, gelatine, corn starch and aerosil. For example, corn starch and microcrystalline can serve as a filling mass and degradants. Highly disperse silicon dioxide (aerosil) acts in turn to make the mass fluent. Gelatine usually serves as an adhesive to get homogeneous granules.

In a preferred embodiment of the present invention the granules constituting said pharmaceutical formulation are covered by an outer phase composed of a mixture comprising starch, particularly com starch, highly dispersed silicon dioxide and magnesium stearate. Such an outer phase properly enables the encapsulation the granules.

A further aspect of the present invention relates to a tablet prepared by using the pharmaceutical formulation according to the present invention. Another aspect of the present invention relates to a capsule filled with said pharmaceutical formulation. Thus, the pharmaceutical formulation according to the present invention can be suitably used as a capsule filling mass. Such a capsule can particularly be a hard gelatine capsule of size 1 or 2 (Ph. Eur.).

In such a capsule according to the present invention the amount of Indibulin as pharmaceutically active ingredient is preferably in the range of about 20 to about 100 mg, more preferably about 30 to about 70 mg, even more preferably about 50 mg per capsule.

A further aspect of the present invention relates to a process for manufacturing said pharmaceutical formulation, comprising the steps of micronizing Indibulin to a particle size of less than 20 µm for more than 99 vol.-% of the particles and homogeneously mixing the micronized Indibulin with at least one hydrophilic surfactant and one or more capsulation excipients. Preferably, the Indibulin is micronized by milling with a jet mill.

In a preferred embodiment of the present invention the micronized Indibulin is homogeneously mixed with com starch, microcrystalline cellulose and aerosil to obtain a powder mixture, while simultaneously gelatine and polysorbate are dissolved in purified water, and subsequently the powder mixture is moistened with the gelatine-polysorbate solution to obtain a homogeneous granulate by sieving through 0.8 mm sieve.

The process according to the present invention can further comprise the step of encapsulating the granules by mixing with an outer phase forming mixture which in turn is obtained by mixing com starch, aerosil and magnesium stearate.

Moreover, the process according to the present invention can further comprise the step of filling the pharmaceutical formulation in hard gelatine capsules of size 1 or 2 (Ph. Eur.) or, alternatively, the pharmaceutical formulation is subsequently processed for tabletting.

### The figures show:

Figure 1 shows the result of a bioavailability study in humans by treating with a formulation according to the present invention first under fasted and for second treatment under fed conditions afterwards (cf. Example 6 hereinbelow).

Figure 2 shows data of said bioavailability study from a patient who was first treated fed and afterwards treated under fasted conditions (cf. Example 6 hereinbelow).

Figure 3 shows the plasma level from 5 patients treated either with the pharmaceutical formulation according to the present invention as obtained in Example 1 hereinbelow or the drinking solution (Example 4) for comparison (cf. Example 7 hereinbelow).

### Examples

The invention is described in the following examples in more detail, but without being limited to those.

### Example 1: Capsule formulation with a strength of 50 mg Indibulin

In order to increase the specific surface of the drug substance Indibulin it is milled via a jet mill. The resulting particle size should be less than 10 µm for more than 90% (volume) of the particles with an average size of about 2 to 4 µm.

The micronized Indibulin is homogeneously mixed with corn starch, microcrystalline cellulose and Aerosil. In parallel gelatine and polysorbate is dissolved in purified water. The powder mixture is then moistened with the gelatine-polysorbate-solution in order to get a homogeneous granulate by sieving through 0.8 mm sieve.

To enable encapsulation the granula is mixed with an outer phase of the capsule mass which is obtained by mixing com starch, Aerosil and Mg-stearate.

The completed capsule filling mass is then filled in hard gelatine capsules of size 2 (Ph. Eur.)

| Composition per unit (Capsule) | | |
|---|---|---|
| Granulate | | |
| | | |
| | Indibulin | 50.0 mg |
| | corn starch | 40.0 mg |
| | aerosil | 3.0 mg |
| | gelatine | 2.5 mg |
| | polysorbate 80 | 5.0 mg |
| | microcristalline cellulose | 45.0 mg |
| | purified water (USP, EP) | q.s. |
| | | |

| Outer phase | | |
|---|---|---|
| | | |
| | corn starch | 10.0 mg |
| | aerosil | 2.5 mg |
| | Mg stearate | 2.0 mg |
| | | |
| | hard gelatine capsules of size 2 | 1 |

### Example 2: Capsule formulation with a strength of 100 mg Indibulin

The manufacturing of a 100 mg strength of Indibulin capsules follows the description in Example 1, but having a slightly different composition per unit.

| Composition per unit (Capsule) | | |
|---|---|---|
| Granulate | | |
| | | |
| | Indibulin | 100.0 mg |
| | com starch | 80.0 mg |
| | aerosil | 6.0 mg |
| | gelatine | 5.0 mg |
| | polysorbate 80 | 10.0 mg |
| | microcristalline cellulose | 90.0 mg |
| | purified water (USP, EP) | q.s. |
| | | |

| Outer phase | | |
|---|---|---|
| | | |
| | corn starch | 20.0 mg |
| | aerosil | 5.0 mg |
| | Mg stearate | 4.0 mg |
| | | |
| | hard gelatine capsules of size 1 | 1 |

### Example 3: Capsule formulation with a strength of 50 mg Indibulin using a poloxamer

| |
|---|
| Composition per unit (Capsule) |

| Granulate | | |
|---|---|---|
| | | |
| | Indibulin | 50.0 mg |
| | com starch | 40.0 mg |
| | aerosil | 3.0 mg |
| | gelatine | 2.5 mg |
| | poloxamere 188 | 5.0 mg |
| | microcristalline cellulose | 45.0 mg |
| | purified water (USP, EP) | q.s. |
| | | |

| Outer phase | | |
|---|---|---|
| | | |
| | com starch | 10.0 mg |
| | aerosil | 2.5 mg |
| | Mg stearate | 2.0 mg |
| | | |
| | hard gelatine capsules of size 2 | 1 |

### Example 4: Drinking solution of Indibulin in 10% lactic acid (1 mg/ml)

For preparation of the drinking solution, a certain amount of the pure active compound is dissolved in lactic acid 90% (Ph. Eur.). Afterwards the obtained solution is diluted with an aqueous solution of glucose and passion fruit flavour to the applicable volume and concentration. The final solution is oversaturated and therefore only stable for 2 hours. Therefore the drinking solution has to be prepared directly prior to administration.

The applicable formulation contains 60 ml of an aqueous drinking solution of Indibulin with a concentration of 1 mg/ml. Glucose and passion fruit flavour are used to modify the taste to make swalling easier.

| Composition of the solution: | |
|---|---|
| Indibulin | 60.0 mg |
| lactic acid 90% | 7269.2 mg |
| glucose Ph.Eur. | 5532.5 mg |
| passion fruit flavour | 96.9 mg |
| pur. water | 50503.7 mg |

### Example 5: Bioavailability studies on animal

Pharmacokinetic studies were carried out in Cynomolgus monkeys, comparing the bioavailability of Indibulin from three different formulation for oral administration and for reference from an intravenously administered solution of Indibulin in solutol^{®}/propane diol:
1. formulation according to the present invention as obtained in Example 1 (50 mg)
2. standard capsule of micronized Indibulin (50 mg)
3. drinking solution of Indibulin in 10% lactic acid, as described in Example 4
4. intravenously administered solution of Indibulin in solutol^{®}/propane diol.

The results show a significant improved bioavailability for the formulation according to the present invention compared with an ordinary capsule formulation. In comparison with the drinking solution containing lactic acid, the bioavailability from the formulation according to the present invention as obtained in Example 1 is lower, but this is compensated by the better tolerability and the higher possible dosing as exemplified by said Example 1; cf. Table 1 hereinbelow (AUC = area under curve).

**Table 1:**

| Meanₐᵣ±SD (n =6) | | | | | | |
|---|---|---|---|---|---|---|
| Admin. Route | Treatment | Animal group | AUC₀₋₂₄* [ng·h/ml] | AUC_{0-24, nom}* [ng·h/ml] | AUC₀₋₃₆ * [ng·h/ml] | AUC_{0-36, norm}* [ng·h/ml] |
| perorally | formulation according to the present invention as obtained in Example 1 (50 mg) | 1a | 524 ± 628 | 429 ± 473 | 561 ± 695 | 455 ± 510 |
| perorally | standard caps (50 mg) | 1b | 76.6 ± 114 | 82.1 ± 139 | 103 ± 113 | 109 ± 137 |
| perorally | solution (10 mg/kg) in 10% lactic acid | 1a | 1886 ± 1085 | 1886 ± 1085 | 2863 ± 1810 | 2863 ± 1810 |
| intravenously | solution (0.2 mg/kg) in sol/prop * | 1b | 299 ± 85.4* | 14949 ± 4270* | - | - |

| | | | | | | |
|---|---|---|---|---|---|---|
| * Plasma samples from intravenously administered animals were only withdrawn until 4 hours and, thus, only AUC₀₋₄ could be calculated | | | | | | |

### Example 6: Bioavailability studies in humans

The formulation of Example 1 was tested in Phase I studies in humans. Patients were treated with the Indibulin capsules under fed and fasted conditions to evaluate the influence of administration prior or after a meal.

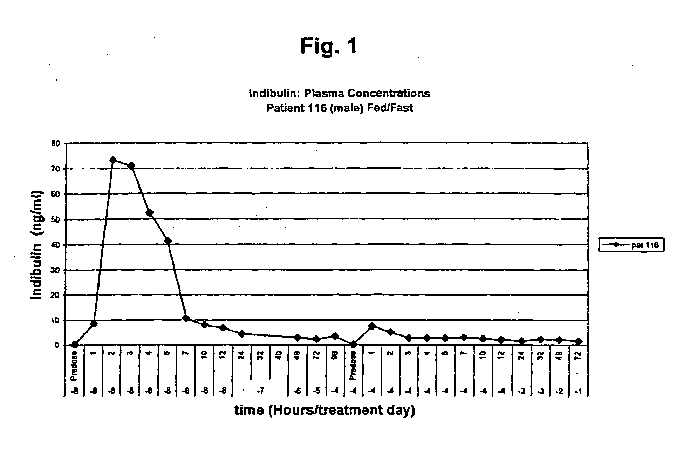

To obtain relevant plasma levels it seems to be better to administer the capsules under fed conditions. Fig. 1 shows treatment first under fed and for second treatment under fasted conditions afterwards. Good bioavailability can be observed in the first treatment whereas after second treatment no plasma level was found.

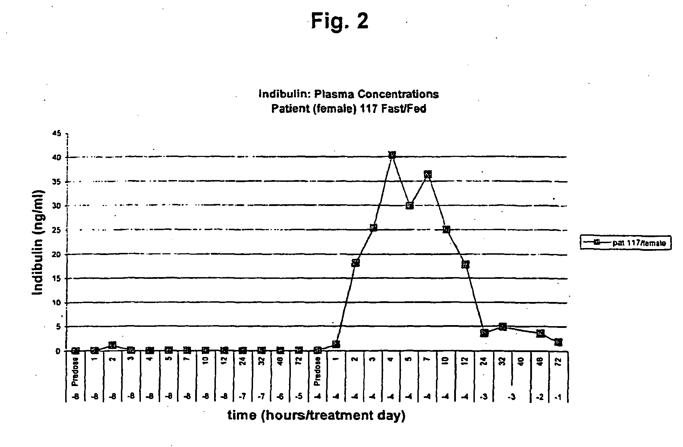

Fig. 2 shows data from a patient who was first treated fasted and afterwards treated under fed conditions. Again, if patient was fasted, no plasma level of Indibulin can be found, but under fed conditions significant plasma levels were observed.

### Example 7: Comparison of the bioavailability of Example 1 (capsule formulation according to the present invention) and Example 4 (drinking solution) in Phase I studies in humans

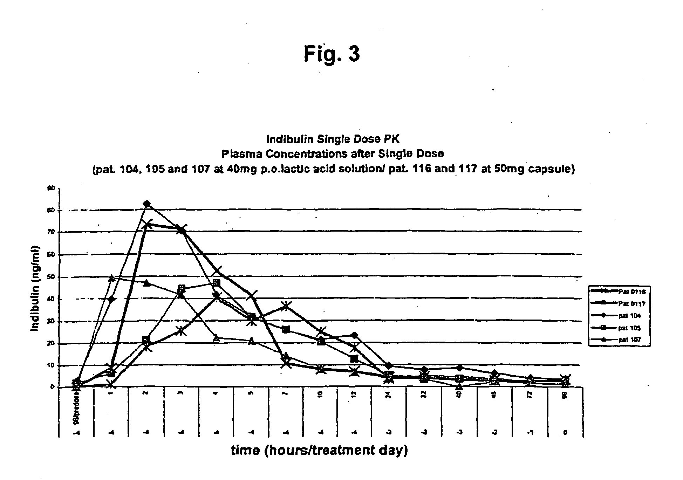

Fig. 3 shows the plasma levels of Indibulin from three patients (patients 104, 105 and 107) treated with 40 mg via the lactic acid drinking solution *versus* two patients (patients 116 and 117) treated with 50 mg via the capsule formulation according to the present invention.

The plasma levels of both formulation were within the same range taking the standard deviation into account, therefore no significant differences can be found. The bioavailability can be stated to be similar for both formulations.

## Claims

1. A pharmaceutical formulation of Indibulin for oral administration comprising a granulate containing micronized Indibulin having a particle size of less than 20 µm for at least 99 vol.-% of the particles, at least one hydrophilic surfactant, and one or more capsulation excipients.

2. The pharmaceutical formulation according to claim 1, wherein the micronized Indibulin has a particle size of less than 10 µm for at least 90 vol.-% of the particles.

3. The pharmaceutical formulation according to claim 1, wherein the micronized Indibulin has a particle size of less than 10 µm for at least 99 vol.-% of the particles.

4. The pharmaceutical formulation according to claim 1, wherein the micronized Indibulin has a mean particle size in the range of 2 to 4 µm.

5. The pharmaceutical formulation according to claim 1, comprising Indibulin in an amount of about 10 to about 50 weight %, the at least one hydrophilic surfactant in an amount of about 1 to about 10 weight %, and the additional capsulation excipients in an amount of about 40 to about 80 weight %.

6. The pharmaceutical formulation according to claim 1, wherein the hydrophilic surfactant is selected from the group consisting of polysorbates, poloxamers, cremophors and polyalkylene glycols.

7. The pharmaceutical formulation according to claim 6, wherein the polysorbate is selected from polysorbate 20, polysorbate 40, polysorbate 60 or polysorbate 80, in particular polysorbate 80.

8. The pharmaceutical formulation according to claim 1, wherein the capsulation excipient comprises at least one selected from the group consisting of cellulose and a derivative thereof, gelatine, starch, particularly com starch, and highly disperse silicon dioxide as well as a mixture thereof.

9. The pharmaceutical formulation according to claim 1, wherein the granules constituting said pharmaceutical formulation are covered by an outer phase composed of a mixture comprising starch, particularly corn starch, highly dispersed silicon dioxide and magnesium stearate.

10. A tablet prepared by using the pharmaceutical formulation as defined in claim 1.

11. A capsule filled with the pharmaceutical formulation as defined in claim 1.

12. The capsule according to claim 11 which is a hard gelatine capsule of size 1 or 2.

13. A capsule according to claim 12 wherein the amount of Indibulin as pharmaceutically active ingredient is in the range of about 20 to about 100 mg, preferably about 30 to about 70 mg, more preferably about 50 mg per capsule.

14. A process for manufacturing the pharmaceutical formulation according to claim 1, comprising the steps of micronizing Indibulin to a particle size of less than 20 µm for more than 99 vol.-% of the particles and homogeneously mixing the micronized Indibulin with at least one hydrophilic surfactant and one or more capsulation excipients.

15. The process according to claim 14, wherein the Indibulin is micronized by milling with a jet mill.

16. The process according to claim 14, wherein the micronized Indibulin is homogeneously mixed with com starch, microcrystalline cellulose and aerosil to obtain a powder mixture, while simultaneously gelatine and polysorbate are dissolved in purified water, and subsequently the powder mixture is moistened with the gelatine-polysorbate solution to obtain homogeneous granules by sieving through 0.8 mm sieve.

17. The process according to claim 14, further comprising the step of encapsulating the granules by mixing with an outer phase forming mixture which in turn is obtained by mixing com starch, aerosil and magnesium stearate.

18. The process according to claim 14, further comprising the step of filling the pharmaceutical formulation in hard gelatine capsules of size 1 or 2.

19. The process according to claim 14, wherein the pharmaceutical formulation is processed for tabletting.

## Patentansprüche

1. Pharmazeutische Formulierung von Indibulin zur oralen Verabreichung, umfassend ein Granulat, enthaltend feinstzerkleinertes Indibulin mit einer Teilchengröße von weniger als 20 µm für mindestens 99 Vol.-% der Teilchen, mindestens ein hydrophiles grenzflächenaktives Mittel und ein oder mehrere Einkapselungsexzipienten.

2. Pharmazeutische Formulierung gemäß Anspruch 1, wobei das feinstzerkleinerte Indibulin eine Teilchengröße von weniger als 10 µm für mindestens 90 Vol.-% der Teilchen aufweist.

3. Pharmazeutische Formulierung gemäß Anspruch 1, wobei das feinstzerkleinerte Indibulin eine Teilchengröße von weniger als 10 µm für mindestens 99 Vol.-% der Teilchen aufweist.

4. Pharmazeutische Formulierung gemäß Anspruch 1, wobei das feinstzerkleinerte Indibulin eine mittlere Teilchengröße in dem Bereich von 2 bis 4 µm aufweist.

5. Pharmazeutische Formulierung gemäß Anspruch 1, umfassend Indibulin in einer Menge von etwa 10 bis etwa 50 Gew.-%, das mindestens eine hydrophile grenzflächenaktive Mittel in einer Menge von etwa 1 bis etwa 10 Gew.-% und die zusätzlichen Einkapselungsexzipienten in einer Menge von etwa 40 bis etwa 80 Gew.-%.

6. Pharmazeutische Formulierung gemäß Anspruch 1, wobei das hydrophile grenzflächenaktive Mittel aus der Gruppe, bestehend aus Polysorbaten, Poloxameren, Cremophoren und Polyalkylenglykolen, ausgewählt ist.

7. Pharmazeutische Formulierung gemäß Anspruch 6, wobei das Polysorbat aus Polysorbat 20, Polysorbat 40, Polysorbat 60 oder Polysorbat 80, insbesondere Polysorbat 80, ausgewählt ist.

8. Pharmazeutische Formulierung gemäß Anspruch 1, wobei der Einkapselungsexzipient mindestens einen, ausgewählt aus der Gruppe, bestehend aus Cellulose und einem Derivat davon, Gelatine, Stärke, insbesondere Maisstärke, und hochdispersem Siliciumdioxid sowie einem Gemisch davon, umfaßt.

9. Pharmazeutische Formulierung gemäß Anspruch 1, wobei die Granalien, welche die pharmazeutische Formulierung aufbauen, durch eine äußere Phase, aufgebaut aus einem Gemisch, umfassend Stärke, insbesondere Maisstärke, hochdisperses Siliciumdioxid und Magnesiumstearat, bedeckt sind.

10. Tablette, hergestellt unter Verwendung der pharmazeutischen Formulierung, wie in Anspruch 1 definiert.

11. Kapsel, gefüllt mit der pharmazeutischen Formulierung, wie in Anspruch 1 definiert.

12. Kapsel gemäß Anspruch 11, welche eine Gelatinehartkapsel der Größe 1 oder 2 ist.

13. Kapsel gemäß Anspruch 12, wobei die Menge an Indibulin als pharmazeutisch wirksamer Bestandteil im Bereich von etwa 20 bis etwa 100 mg, vorzugsweise etwa 30 bis etwa 70 mg, mehr bevorzugt etwa 50 mg per Kapsel, ist.

14. Verfahren zur Herstellung der pharmazeutischen Formulierung gemäß Anspruch 1, umfassend die Schritte des Feinstzerkleinerns von Indibulin zu einer Teilchengröße von weniger als 20 µm für mehr als 99 Vol.-% der Teilchen und des homogenen Mischens des feinstzerkleinerten Indibulins mit mindestens einem hydrophilen grenzflächenaktiven Mittel und einem oder mehreren Einkapselungsexzipienten.

15. Verfahren gemäß Anspruch 14, wobei das Indibulin durch Mahlen mit einer Strahlmühle feinstzerkleinert wird.

16. Verfahren gemäß Anspruch 14, wobei das feinstzerkleinerte Indibulin homogen mit Maisstärke, mikrokristalliner Cellulose und Aerosil homogen gemischt wird, um ein Pulvergemisch zu erhalten, während gleichzeitig Gelatine und Polysorbat in gereinigtem Wasser gelöst werden, und nachfolgend das Pulvergemisch mit der Gelatine-Polysorbat-Lösung befeuchtet wird, um homogene Granalien durch Sieben durch 0,8 mm Sieb zu erhalten.

17. Verfahren gemäß Anspruch 14, weiter umfassend den Schritt des Einkapselns der Granalien durch Mischen mit einem Gemisch, das eine äußere Phase bildet, welches wiederum durch Mischen von Maisstärke, Aerosil und Magnesiumstearat erhalten wird.

18. Verfahren gemäß Anspruch 14, weiter umfassend den Schritt des Füllens der pharmazeutischen Formulierung in Gelatinehartkapseln der Größe 1 oder 2.

19. Verfahren gemäß Anspruch 14, wobei die pharmazeutische Formulierung durch Tablettieren verarbeitet wird.

## Revendications

1. Formulation pharmaceutique d'indibuline pour une administration orale, comprenant des granules contenant de l'indibuline micronisée ayant une taille des particules inférieure à 20 µm pour au moins 99 % en volume des particules, au moins un agent tensioactif hydrophile et un ou plusieurs excipients d'encapsulation.

2. Formulation pharmaceutique selon la revendication 1, dans laquelle l'indibuline micronisée a une taille des particules inférieure à 10 µm pour au moins 90 % en volume des particules.

3. Formulation pharmaceutique selon la revendication 1, dans laquelle l'indibuline micronisée a une taille des particules inférieure à 10 µm pour au moins 99 % en volume des particules.

4. Formulation pharmaceutique selon la revendication 1, dans laquelle l'indibuline micronisée a une taille moyenne des particules dans la plage de 2 à 4 µm.

5. Formulation pharmaceutique selon la revendication 1, comprenant de l'indibuline en une quantité d'environ 10 à environ 50 % en poids, l'agent tensioactif hydrophile, au nombre d'au moins un, en une quantité d'environ 1 à environ 10 % en poids, et les excipients supplémentaires d'encapsulation en une quantité d'environ 40 à environ 80 % en poids.

6. Formulation pharmaceutique selon la revendication 1, dans laquelle l'agent tensioactif hydrophile est choisi dans l'ensemble constitué de polysorbates, poloxamères, crémophores et polyalkylèneglycols.

7. Formulation pharmaceutique selon la revendication 6, dans laquelle le polysorbate est choisi parmi le polysorbate 20, le polysorbate 40, le polysorbate 60 ou le polysorbate 80, en particulier le polysorbate 80.

8. Formulation pharmaceutique selon la revendication 1, dans laquelle l'excipient d'encapsulation comprend au moins un agent choisi dans l'ensemble constitué de la cellulose et d'un dérivé de celle-ci, de la gélatine, de l'amidon, en particulier l'amidon de maïs, et du dioxyde de silicium très dispersé, ainsi que d'un mélange de ceux-ci.

9. Formulation pharmaceutique selon la revendication 1, dans laquelle les granules constituant ladite formulation pharmaceutique sont recouverts d'une phase externe composée d'un mélange comprenant de l'amidon, en particulier de l'amidon de maïs, du dioxyde de silicium très dispersé et du stéarate de magnésium.

10. Comprimé préparé en utilisant la formulation pharmaceutique telle que définie dans la revendication 1.

11. Capsule remplie de la formulation pharmaceutique telle que définie dans la revendication 1.

12. Capsule selon la revendication 11, qui est une capsule de gélatine dure de taille 1 ou 2.

13. Capsule selon la revendication 12, dans laquelle la quantité d'indibuline en tant qu'ingrédient pharmaceutiquement actif est dans la plage d'environ 20 à environ 100 mg, de préférence d'environ 30 à environ 70 mg, de façon plus préférée d'environ 50 mg par capsule.

14. Procédé de fabrication de la formulation pharmaceutique selon la revendication 1, comprenant les étapes de micronisation d'indibuline jusqu'à une taille des particules inférieure à 20 µm pour plus de 99 % en volume des particules et le mélange homogène de l'indibuline micronisée avec au moins un agent tensioactif et un ou plusieurs excipients d'encapsulation.

15. Procédé selon la revendication 14, dans lequel l'indibuline est micronisée par broyage avec un broyeur à jet.

16. Procédé selon la revendication 14, dans lequel on mélange l'indibuline micronisée de façon homogène avec de l'amidon de maïs, de la cellulose microcristalline et de l'aérosil pour obtenir un mélange pulvérulent tout en dissolvant simultanément de la gélatine et du polysorbate dans de l'eau purifiée, et ensuite on humidifie le mélange avec la solution de gélatine et de polysorbate pour obtenir des granules homogènes par tamisage à travers un tamis de 0,8 mm.

17. Procédé selon la revendication 14, comprenant en outre l'étape d'encapsulation des granules par mélange avec un mélange formant une phase externe, qu'on obtient à son tour en mélangeant de l'amidon de maïs, de l'aérosil et du stéarate de magnésium.

18. Procédé selon la revendication 14, comprenant en outre l'étape d'introduction de la formulation pharmaceutique dans des capsules de gélatine dure de taille 1 ou 2.

19. Procédé selon la revendication 14, dans lequel on transforme la formulation pharmaceutique pour la fabrication de comprimés.
